Europäisches Patentamt

European Patent Office

Office européen des brevets

(11) Publication number: **0 402 402 B1**

(19)

# EUROPEAN PATENT SPECIFICATION

(45) Date of publication of patent specification: **27.12.95**  (51) Int. Cl.⁶: **C12N 15/11**, A61K 31/70

(21) Application number: **89903415.1**

(22) Date of filing: **24.02.89**

(86) International application number:
**PCT/US89/00752**

(87) International publication number:
**WO 89/08146 (08.09.89 89/21)**

(54) **INHIBITION OF HTLV-III BY EXOGENOUS OLIGONUCLEOTIDES**

(30) Priority: **26.02.88 US 160574**

(43) Date of publication of application:
**19.12.90 Bulletin 90/51**

(45) Publication of the grant of the patent:
**27.12.95 Bulletin 95/52**

(84) Designated Contracting States:
**AT BE CH DE FR GB IT LI LU NL SE**

(56) References cited:
**EP-A- 0 288 163
EP-A- 0 300 687
WO-A-86/05516
WO-A-87/03451
WO-A-87/07300**

(73) Proprietor: **WORCESTER FOUNDATION FOR
EXPERIMENTAL BIOLOGY
222 Maple Avenue
Shrewsbury, MA 01545 (US)**

(72) Inventor: **GOODCHILD, John
11 Greybert Lane
Worcester, MA 01602 (US)**
Inventor: **ZAMECNIK, Paul, C.
65 Commons Drive
Shrewsbury, MA 01545 (US)**
Inventor: **AGRAWAL, Sudhir
46G Brandywine Drive
Shrewsbury, MA 01545 (US)**

(74) Representative: **Holdcroft, James Gerald, Dr.
et al
Graham Watt & Co.,
Riverhead
Sevenoaks, Kent TN13 2BN (GB)**

**Description**

Background

The Retroviridae virus family includes viruses which contain an RNA genome and an RNA-dependent DNA polymerase activity (reverse transcriptase). During their growth cycle, retroviridae, or retroviruses as they are more commonly called, copy their RNA into proviral DNA. Proviral DNA becomes inserted (integrated) into the chromosomal DNA of the host where it uses the transcriptional and translational machinery of the host to express viral RNA and proteins. Viruses are released from the cell by budding from the cytoplasmic membrane. Most infections do not necessarily kill cells. Rather, infected cells may continue to grow and differentiate while continuously producing virus.

Three major classes of human retroviruses have been identified: 1) T-cell lymphotropic viruses; 2) endogenous genetic elements related to primate C-type viruses; and 3) foamy viruses.

Human T-cell leukemia-lymphotropic virus (HTLV) refers to a family of T cell tropic retroviruses. Such viruses, which have a role in causing certain T cell neoplasms, are generally divided into three main types or subgroups: 1) HTLV-type I (HTLV-I), which appears to cause adult T-cell leukemia-lymphoma (ATLL); 2) HTLV-type II (HTLV-II), which has been isolated from an individual having a T-cell variant of hairy cell leukemia; and 3) HTLV-type III (HTLV-III), which has been identified as the etiologic agent of acquired immune deficiency syndrome (AIDS). HTLV-III is also known as lymphadenopathy-associated virus (LAV), AIDS related virus (ARV) and human immunodeficiency virus (HIV-1). Popovic, M. et al., Science, 224:497-500 (1984); Gallo, R.C. et al., Science, 224:500-503 (1984); Wong-Staal, F. and Gallo, R.C., Nature, 317:395-403 (1985); and Curran, J.W. et al., Science, 229:1352-1357 (1985). More recently, HIV-2 has been discovered by Montagnier in patients who have AIDS but show no signs of HIV-1 infection. Science, 236:390-392 (1987).

AIDS was first recognized in 1981 and since that time, the disease has come to be recognized as a new epidemic. RNA Tumor Viruses (2d edition), Volume 2, pp 437-443, Cold Spring Harbor Laboratory (1985).

Patients with AIDS exhibit clinical manifestations which include severe immunodeficiency which generally involves a depletion of helper T lymphocytes; malignancies; and opportunistic infections. The disease at this time is incurable and the mortality rate among AIDS patients is high.

Because the disease has severe, generally life threatening effects, there is great interest in finding means of protecting the population from it and of treating those who contract it. At the present time, much effort is being put into developing methods of detecting the presence of HTLV-III (HIV-1) in body tissues and fluids (e.g., blood, saliva) and into developing vaccines which will protect recipients from HTLV-III. However, there is no known method which is satisfactory, either for preventing the disease or for treating those who become infected with the virus. In fact, current efforts to develop a broad spectrum anti-HTLV-III vaccine may be seriously compromised, in light of the variation in envelope proteins (which are the principal antigenic determinants of the virus) observed among various strains of HTLV-III. Hahn, G.H. et al., Proceedings of the National Academy of Sciences, USA, 82:4813-4817 (1985); Benn, S. et al., Sciences, 230:949-951 (1985). Other methods of blocking the effects of the virus are clearly needed.

Summary of the Invention

This invention relates to a preparation for use in therapy comprising a single stranded modified oligonucleotide of 8-50 nucleotides in length, wherein the modified oligonucleotide is one which is modified at an internucleoside phosphate, being a phosphomorpholidate, phosphopiperazine or phosphoroamidate derivative, the oligonucleotide being capable of selectively hybridizing with a highly conserved region of the HTLV-III genome to induce selective hybridization arrest of HTLV-III replication and/or gene expression, the highly conserved region comprising any of:

(i) the tRNA$^{lys}$ primer binding site;

(ii) regions of the HTLV-III genome vicinal in the 5' direction to the tRNA$^{lys}$ primer binding site;

(iii) the tRNA$^{lys}$ primer binding site and regions of the HTLV-III genome vicinal in the 5' direction to the tRNA$^{lys}$ primer binding site;

(iv) the mRNA donor splice sites;

(v) the mRNA acceptor splice sites;

(vi) the initiator codon for the gag gene;

(vii) the initiator codon for the env gene;

(viii) the initiator codon for the tat gene;

(ix) the initiator codon for the sor gene;

(x) the initiator codon for the 3' orf gene;

(xi) the cap nucleotide of the HTLV-III genome;

(xii) the art gene or portions thereof;

(xiii) the region of HTLV-III genome encoding a frameshift wherein the modified oligonucleotide is one which is modified at an internucleoside phosphate, being a phosphomorpholidate, phosphopiperazine or phosphoroamidate derivative.

In another aspect, the invention relates to an in vitro method of inhibiting HTLV-III replication, HTLV-III gene expression or both in cells containing HTLV-III, comprising introducing into the cells a modified oligodeoxynucleotide.

In another aspect, the invention relates to use of a preparation (or modified derivative thereof) for the manufacture of a medicament for use in AIDS chemotherapy, e.g. in inhibiting HTLV-III replication and/or gene expression in peripheral human blood cells (e.g. human T lymphocytes) infected with HTLV-III.

The oligonucleotides of this invention, which can be oligodeoxyribonucleotides or oligoribonucleotides, are complementary to regions on the HTLV-III genome which are highly conserved, and whose function is necessary for normal replication or gene expression by HTLV-III. The oligonucleotides can be used to block HTLV-III replication, gene expression or both and thus can be used as chemotherapeutic agents in inhibiting replication and gene expression by the virus. In addition, they can be used to detect the presence of HTLV-III in samples such as blood, urine and saliva.

Oligonucleotides of the present invention are complementary to target sites which are highly conserved regions of the HTLV-III genome. These include the cap site; the primer binding site; nucleotide sequences vicinal to the primer binding site in the 5' direction; mRNA donor splice and acceptor splice sites; the HTLV-III initiator codons, including those for the gag, the sor, the tat, the env, and the 3'ORF sequences; the art gene or a portion thereof; polyadenylation signal; and the region of the genome responsible for the frameshift known to occur during transcription. These oligodeoxynucleotides can be used to inhibit HTLV-III replication and/or gene expression in the HTLV-III infected cells. They can be administered to individuals to block HTLV-III replication and/or gene expression as a means of chemotherapeutic treatment of acquired immune deficiency syndrome (AIDS) and of AIDS related complex (ARC). The method of the present invention is referred to as hybridization arrest.

Use of such oligonucleotides has at least two important advantages. First, the antiviral effects observed are very specific. For example, a specific sequence of 20 nucleotides would not be expected to occur at random more often than about one time in $10^{12}$. There are about $4 \times 10^9$ nucleotide pairs in the human genome and thus, the specificity of a 20-nucleotide sequence chosen from a conserved region of HTLV-III is predicted to be great. Second, the cellular toxicity of the oligonucleotides is also low, in comparison with most nucleoside analogues (e.g., those used in cancer chemotherapy, graft-host immunology and viral inhibition); such analogues are converted into nucleotides, which are subsequently incorporated into cellular DNA.

Oligonucleotides complementary to the same regions of the HTLV-III genome can be used to determine whether HTLV-III is present or absent in a sample such as blood, saliva or urine by determining whether cell death occurs in cells which are normally killed by HTLV-III virus (such as T lymphocytes) when they are cultured with the sample to be tested and whether cell death can be inhibited by the oligonucleotide.

This same approach can also be used to inhibit replication of other retroviruses and other viruses (i.e., DNA viruses, RNA viruses) and, thus, gene expression in host cells. When used to inhibit other retroviruses, the method of the present invention will use oligonucleotides which are complementary to regions of the retroviral genome whose function is necessary for normal replication or gene expression and which are highly conserved will be used. In the case of other viruses, oligonucleotides complementary to viral RNA or DNA are used.

The method of the present invention can also be used to inhibit the activity of other (non-viral) infectious agents, such as bacteria, fungi, protozoa and worms. In these applications, oligonucleotides complementary to specific, essential regions of DNA or RNA of the infectious agent are used.

Brief Description of the Figure

The Figure is a schematic representation of the primary nucleotide sequence of the HTLV-III genome and of the location on the genome of oligonucleotide competitive inhibition targets.

Detailed Description of the Invention

INHIBITION OF HTLV-III

The primary nucleotide sequence of the HTLV-III/LAV genome has been determined by several groups of investigators. Ratner, L. et al., Nature 313:277-284 (1985); Wain-Hobson, S. et al., Cell 40:9-17 (1985); Sanchez-Pescador, R. et al., Science 227:484-492 (1985); Muesing, M.A. et al., Nature 313:450-458 (1985).

The genome of HTLV-III is shown in the Figure. The HTLV-III genome has been shown to be considerably more variable than the genomes of most retroviruses. RNA Tumor Viruses (2d edition) Volume 2, p 446, Cold Spring Harbor Laboratory (1985). Like other retroviruses, HTLV-III has in its genome three genes which encode viral proteins: 1) the gag gene, which encodes nucleocapsid or internal structural proteins of the virus; 2) the pol gene, which encodes reverse transcriptase (an RNA-directed DNA polymerase responsible for transcribing RNA into DNA); and 3) the env gene, which encodes the envelope glycoproteins of the virion. In addition, two other open reading frames are known; one (sor) overlaps with the 3' end of the pol gene and the other (3'ORF), located at the extreme 3' end of the genome, slightly overlaps the env gene and continues through most of the U3 region. The genome has also been shown to contain tat-III and art. Tat-III is the trans-activation gene of HTLV-III; it encodes trans-activator protein, which greatly accelerates viral protein synthesis in infected cells. Art (antirepression of the translation-transactivator gene) has only recently been found in the HTLV-III genome and appears to work cooperatively with tat in producing viral core and envelope proteins.

Other regions of the RNA of HTLV-III are a cap nucleotide, which occurs at the extreme 5' end of the genome; a short sequence (R) which is repeated at both ends of the RNA; a short sequence unique to the 5' end (U5); and a sequence unique to the 3' end (U3) of the RNA. Each of the last three components is present twice in viral DNA; each forms part of the long terminal repeat (LTR) sequence found at both ends of the unintegrated linear DNA product of reverse transcription. The HTLV-III genome also contains a primer binding site (PBS) adjacent to U5 (at its 3' end); the PBS is complementary to the 3' end of tRNA lysine and functions as primer for synthesis of the minus strand of viral DNA. Donor splice (S.D.) and acceptor splice (S.A.) sites are also located on the viral RNA. Donor splice sites are sequences at which a 5' portion of the viral genome is joined to a portion of the 3' end of viral RNA, forming a spliced, subgenomic messenger RNA. Acceptor splice sites are sequences at which portions of the 3' end of viral RNA join donor splice sites to form subgenomic messenger RNA.

As mentioned above, different HTLV-III strains have been reported to have variations in envelope proteins. These variations may compromise the development of a broad spectrum anti-HTLV-III vaccine. In contrast, the primary nucleotide sequence of the primer area and certain other parts of the HTLV-III genome are highly conserved.

It has been now shown that complementary oligodeoxynucleotides directed toward such highly conserved regions of the HTLV-III genome inhibit virus replication and/or gene expression in cultured HTLV-III-transformed human lymphocytes.

Targeted Regions of the HTLV-III Genome

As mentioned, several regions of the HTLV-III genome are highly conserved; these regions or parts thereof can be targeted for inhibition by complementary oligonucleotide sequences. These regions, referred to as oligonucleotide competitive inhibition targets, include: 1) the cap site; 2) sequences of nucleotides 5' to the primer tRNA$^{lys}$ binding site; 3) the primer binding site or a segment thereof; 4) a combination of sequences 5' to the primer tRNA$^{lys}$ binding site and the primer binding site; 5) sequences of the mRNA donor or acceptor splice sites; 6) the initiator codons for the gag, the sor, the tat, the env and the 3'ORF sequences; 7) the art gene or portions thereof; 8) the region of the genome responsible for the frameshift known to occur during transcription; and 9) polyadenylation signal. The location of these regions (except the art gene) is indicated in the Figure; the art gene is located close to the tat gene.

It has been demonstrated that oligodeoxynucleotides complementary to four of the above mentioned highly conserved regions inhibit virus replication or gene expression in cultured HTLV-III-transformed human lymphocytes. That is, oligodeoxynucleotides complementary to 1) sequences 5' to the primary tRNA$^{lys}$ binding site; 2) the primer binding site; 3) sequences of a mRNA donor splice site; or 4) sequences of a mRNA acceptor splice site have been shown to cause inhibition. In general, any highly conserved region of the HTLV-III genome which encodes information necessary for viral replication or gene expression (e.g., protein synthesis) is a potential target for complementary oligodeoxynucleotides.

Complementary Oligonucleotide Sequences

The oligonucleotide sequences complementary to the competitive inhibition targets can be oligoribonucleotide sequences or oligodeoxyribonucleotide sequences. Both types are referred to herein as oligonucleotides and, alternatively, as hybridons. As described here, the oligonucleotides were synthesized on an automated DNA synthesizer. It is possible, however, to produce the desired sequences by using genetically engineered organisms, such as bacteria or viruses.

Oligodeoxynucleotide sequences of varying lengths were used to assess their inhibitory effect on viral replication and gene expression. For example, several nucleotide sequences complementary either to nucleotide sequences of the HTLV-III genome which are 5' to the primer tRNA$^{lys}$ binding site or to nucleotide sequences which straddle the primer binding site and the adjacent region (in the 5' direction) were synthesized and their inhibitory effects tested. As described in greater detail in Example 3, a 12-nucleotide sequence (mer), a 20-nucleotide sequence and a 26-nucleotide sequence have been made and their inhibitory effects on viral replication and gene expression measured. The 12-nucleotide and the 20-nucleotide sequences are complementary to portions of the HTLV-III genome close to the primer binding site, in the 5' direction. The 26-nucleotide sequence is complementary to the primer binding site; it has been shown to be particularly effective in hybridizing to viral targets and in inhibiting the virus.

In addition, oligodeoxynucleotide sequences complementary to splice donor or splice acceptor sites of HTLV-III mRNA have been made and their inhibitory effects assessed. In particular, a 20-nucleotide sequence complementary to a splice donor site from the 3'-open reading frame region (See the Figure) and two 20-nucleotide sequence complementary to the a-1 and a-1' splice acceptor sites, the former necessary for the production of transactivating factor, have been synthesized and their inhibitory effects measured.

Viral replication was assayed as reverse transcriptase activity level, gene expression as production of viral proteins p15 and p24 and inhibition of syncytial formation. Inhibition of viral replication is reflected in reduced reverse transcriptase activity levels; inhibition of viral gene expression is indicated by reduction in viral protein production (p15 and p17 as used herein are two designations of the same viral protein). As shown in Table 1, HTLV-III replication and protein expression were inhibited in almost every instance. The greatest inhibitory effect was evident when the 20-nucleotide sequence complementary to the splice acceptor site was tested on cultures of HTLV-III infected cells.

As described in detail in Example 4, a variety of oligomers of the same nucleotide sequence (either 15 mers or 20 mers), which are unmodified oligomers; oligomers with all internucleoside phosphates modified as phosphorothioates, methylphosphonates or phosphoromorpholidates; oligomers with replacement of just 3' and 5' terminal and penultimate internucleoside phosphates by the above-mentioned modifications; or oligomers with blocking groups at 5' and/or 3' terminal hydroxyl groups have been compared for their effects on growth and expression of HIV. As described in Example 4 and as shown in Tables 3-7, a variety of sites within the HIV genome were targeted. Results showed inhibition of up to 80-95% of the functions assayed (i.e., viral reverse transcriptase, p17 and p24 viral protein synthesis, syncytial formation and growth of host cells), although considerable variability, as described in Example 4, was observed.

Other complementary oligonucleotide sequences which can be used are determined by the competitive inhibition target(s) selected. Oligonucleotide sequences can be complementary to a single competitive inhibition target or can contain sequences complementary to more than one such target. For example, oligomers can be produced which are complementary to the HTLV-III primer binding site and the region of the genome immediately adjacent to that site in the 5' direction; to two splice donor sites; to two splice acceptor sites; or to any combination of competitive inhibition targets.

Other characteristics of the oligonucleotides used to inhibit viral processes include their length; their modification and the location of groups used to modify them. For example, the length of the oligonucleotides to be used will be determined by factors such as the desired specificity of inhibition, size necessary to block viral function, and effect on transmembrane passage. For example, the work described herein has made use of complementary oligodeoxynucleotides ranging in length from 14 to 26 nucleotides. However, there is potentially no limit to the length of the oligonucleotides to be used and length must be determined carefully, in light of the fact it plays a role in the nucleic acid hybrid's chemical stability and in the viral inhibition achieved. Generally, oligonucleotides used to inhibit HTLV-III will be 8-50 nucleotides in length.

Oligonucleotides to be used can be modified at a variety of locations along their length. For example, they can be modified by the addition of groups at the 5' end (i.e., a dideoxy group), the 3' end or both (i.e., an isourea group), as well as on the internal phosphate groups (i.e., thiophosphates, morpholidates, alkylaminophosphates, piperazine phosphates) or on the bases. Some of these modifications are described above and in Example 4 and Tables 3-7. Whether oligonucleotides to be used are modified and, if so, the location of the modification(s) will be determined, for example, by the desired effect on viral activity (e.g., inhibition of viral replication, gene expression or both), uptake into infected cells, inhibition of degradation of the oligonucleotides once they are inside cells, and prevention of their use as a primer by reverse transcriptase. For example, in order to inhibit reverse transcriptase activity (and thus viral replication) it may be necessary to block the 3' end of a sequence complementary to the primer binding site and/or sequences vicinal to the primer binding site in the 5' direction (for example, by a 2'3' dideoxynucleotide). In this way, the oligonucleotide complementary to either or both of those regions cannot itself serve as a template for transcriptase activity.

If the desired effect is increased uptake of the oligonucleotide into infected cells, modification of the oligonucleotide by addition at the 5' end of a lipophilic group or an agent known to enhance uptake, such as polylysine or polyarginine would be beneficial. Modification of oligonucleotides can also be carried out by the addition of an intercalating agent (e.g., acridine dye) at 5' or 3' termini, on bases, or on internucleophosphate groups. Modification in this manner may result in stronger binding between the oligonucleotides and the HTLV-III nucleic acids. Asseline, U. et al., C.R. Acad. Sc. Paris, 369-372 (1983).

It would be particularly desirable to permanently modify the target site by attaching to the hybridon chemically reactive groups capable of cross linking, cleaving or otherwise modifying the target site. As shown in Table 1, the 12 nucleotide sequences complementary to the region of the HTLV-III genome 5' to the primer binding site were blocked at the 3' end by ddT. Early work on Rous sarcoma virus inhibition indicates that the 5' and 3' end blocked hybridon was a more effective inhibitor than an unblocked hybridon. A hybridon is defined as an oligonucleotide complementary to single-stranded DNA or RNA, which modulates the function of the DNA or RNA by competitive hybridization. Zamecnik, P. and M.L. Stephenson, Proceedings of the National Academy of Sciences, USA, 75:280284 (1978).

Chain terminator(s) to be used in modifying oligonucleotides for use in inhibiting viral replication and gene expression can be, for example, ddT (as described above and in Example 3), the isourea group, the dimethoxytrityl group, or, in fact, any 3' modified function. Selection of the chain terminator is based, for example, on the absence of a 3' OH group (which can act as a substrate for reverse transcriptase); lack of or low cellular toxicity; lipophilicity; and lack of impairment of hydrogen bonding properties of the oligonucleotide.

It has now been demonstrated that 3' or 5' exonucleases, such as phosphodiesterases from snake venom or spleen, can progress past a single methylphosphonate internucleoside linkage. It has also been shown that two such linkages in succession constitute a strong block to the enzyme's activity and can increase the half life of an oligomer in the presence of such an enzyme by 100 fold. Good protection from destruction of an oligodeoxynucleotide by exonucleases can be afforded by using methylphosphonate linkages at the last two positions at each end of the molecule. This enhances the survival of the compound in vivo.

Another modification known to inhibit nuclease digestion is the replacement of internucleoside phosphate by thiophosphate. Matzura, H. and F. Eckstein, European Journal of Biochemistry, 3:448 (1968). Thus, some or all of the phosphates in an oligonucleotide sequence can be replaced by thiophosphate to suppress nucleolytic degradation.

Internucleoside phosphoromorpholidate and phosphoropiperazine derivatives have been shown to be resistant to nucleases and are inhibitory to HIV replication and expression. It is not necessary for an oligonucleotide to be non-ionic in order to enter cells. Therefore, oligonucleotides with a variety of oligonucleotides with mixed ionic and non-ionic internucleoside linkages will hybridize effectively with the genome target and, in addition, have particular advantages over the unmodified oligomer. For example, they have greater resistance to cellular nucleases than do unmodified oligomers and result in different distributions within the cell than the unmodified oligomers. Such properties may enhance to therapeutic efficacy.

INHIBITION OF HTLV-III-INFECTED CELLS

Using the oligodeoxynucleotide sequences described above and in Examples 3 and 4, HTLV-III replication and gene expression are inhibited in HTLV-III-infected cells in tissue culture. The oligodeoxynucleotides described were added to peripheral human blood cells (PB) infected with HTLV-III and to transformed T-lymphocyte (H9) cells infected with HTLV-III. The oligodeoxynucleotide was usually added at time zero only and observation of inhibitory effects was made at 96 hours. In one case, the oligonucleotide was added to fresh culture medium daily for 3 days.

As described in Example 3, reverse transcriptase activity and viral p15 and p24 protein production were used as indicators of inhibition of HTLV-III replication and gene expression, respectively. As shown in Table 1 and described in detail in Example 3, inhibition was greatest when a 20-nucleotide sequence complementary to a splice acceptor site was added to HTLV-III-infected transformed T-lymphocytes. Inhibition was observed under essentially all experimental conditions (see Table 1).

Important considerations in this context are the concentration at which the complementary oligodeoxynucleotides are applied and the timing (scheduling) of their administration. As shown in Table 1, the oligodeoxynucleotides were added at concentrations ranging from 5 to 50 ug/ml. of culture medium. These concentrations were generally effective in producing an inhibitory effect but this range is by no means to be considered limiting. As described, the oligodeoxynucleotide was usually added at one time only; it seems, however, that daily addition (or more frequent addition) is more effective than a single dose. H9 cells infected for 4 days with HIV were also treated at that point with hybridons and inhibition of p17 and p24 protein synthesis was observed.

As described in Example 4, growth and expression of HIV in suspension tissue cultures of H9 cells were monitored by assay of viral reverse transcriptase p17 and p24 viral protein synthesis, syncytial formation and growth of host cells. Inhibition observed with the oligomers described was as high as 80-95% with initial concentrations of 10-100 ug per ml. or all of the classes of oligomers.

## INHIBITION OF HTLV-III IN HUMANS

Based on the information gained from inhibition of HTLV-III-infected cells in tissue culture, it is possible to formulate a strategy for similar inhibition of HTLV-III in AIDS patients, as well as in individuals carrying the AIDS virus but not manifesting symptoms of the disease.

The strategy used in treating a particular individual depends on the status of the individual and the objective of the treatment. That is, an individual who has been found to be carrying the HTLV-III virus but shows no symptoms of AIDS might be treated differently, in terms of both the type of oligonucleotide(s) administered and the dose given, than an individual who does, in fact, have AIDS. In addition, treatment might well differ if its objective is to protect uninfected cells or to have an effect on cells which are already infected.

For example, an individual known to be harboring the virus but yet manifesting no sign of AIDS could be given a long-term or intermittent dosage schedule of oligonucleotides whose inhibitory effects stop reverse transcription (e.g., oligonucleotides complementary to the primer binding site and/or sequences close to the primer binding site in the 5' direction). In this way, the first step in viral life or replication is inhibited because viral DNA cannot be made and the virus is unable to proliferate. However, in an AIDS patient, cells are already infected and treatment must inhibit expression of genes (viral DNA) already present in the infected cells. In this case, oligonucleotides complementary to, for example, initiator codons for genes encoding viral proteins, are required to prevent viral construction. In an AIDS patient, uninfected cells can also be protected by administration of oligonucleotides capable of blocking reverse transcription.

In any treatment situation, however, oligonucleotides must be administered to individuals in a manner capable of getting the oligonucleotides initially into the blood stream and subsequently into cells. Alternatively, oligonucleotides resistant to nucleases by internucleotide phosphate modifications might be taken orally in capsule form. As a result, the oligonucleotides can have the desired effects: getting into HTLV-III infected cells to slow down or prevent viral replication and/or into as yet uninfected cells to provide protection.

Oligonucleotides whose presence in cells can stop reverse transcription and oligonucleotides whose presence in cells can inhibit protein synthesis can be administered by intravenous injection, intravenous drip or orally. The dose to be administered varies with such factors as the size and age of the patient, stage of the disease and the type of oligonucleotide to be given.

## Detection of the HTLV-III Virus in Samples

The oligonucleotide sequences of the present invention can also be used in determining whether the HTLV-III virus is present or absent in samples such as blood, urine, saliva and tears. An aliquot of the sample to be analyzed is added to a culture of cells which are normally killed by the HTLV-III virus (e.g., T lymphocytes); this is the control. A second aliquot is added to a separate culture of T lymphocytes, along with oligonucleotides complementary to one or more of the regions of the HTLV-III genome describe above; this is the test sample. Both cultures are maintained under conditions appropriate for growth and subsequently analyzed (e.g., visually/microscopically) for growth of the T lymphocytes. If the HTLV-III virus is present, the T lymphocytes in the control sample will be killed; if not, the T lymphocytes survive. T lymphocytes in the test sample, however, will continue to be viable because of the protection provided by the complementary oligonucleotides included in the culture. Visual comparison of the two samples makes it possible to determine whether HTLV-III virus is present or absent in each.

INHIBITION OF RETROVIRUSES OTHER THAN HTLV-III

As explained above, it is also possible to use oligonucleotides and methods of the present invention to inhibit replication of retroviruses other than HTLV-III. In these applications, oligonucleotide sequences complementary to regions of the retroviral genome whose function is necessary for normal retroviral replication or gene expression and which are highly conserved will be used.

For example, other retroviruses which cause disease in humans are HTLV-I and HTLV-II (which cause leukemia) and HIV-2 (which causes AIDS).

Oligomers complementary to regions of HTLV-I RNA have been made and shown to inhibit viral replication through the use of assays similar to those described for HTLV-III (Example 3). The sequences used for this work are the following:

1. 5'-AGA AGG CGA AAC AGC AT AGT
2. 5'-GGG CTG ATA ATA AGC ATG GT
3. 5'-GCC GAT AAC GCG TCC ATC GA
4. 5'-GGG GAG TAT TTG CGC ATG GC
5. 5'-ACT GTG TAG TAA ATT
6. 5'-CCC CAA CTG TGT ACT

Oligonucleotides 1-4 are complementary to putative protein initiation sites in the so called 'X' region near the 3' end of HTLV-I. Oligonucleotides 5 and 6 are complementary to the primer binding site region. (See Seiki, M. et al., Proceedings of the National Academy of Sciences, USA, 80:3618 (1983) for HTLV-I sequence).

All of these were shown to be inhibitory to HTLV-I. It is also likely that splice sites, the primer binding site, the cap site, the polyadenylation signal, packaging signal and protein sites would be targets for hybridization arrest.

HIV-2 has been shown to be similar in overall structure and biological effect to HIV-1, although there are considerable differences in nucleotide sequence (M. Guyader, et al., Nature, 326:662 (1987)). Analogous target sites for the action of oligonucleotides exist in HIV-2 as occur in HIV-1. It appears that only the primer binding site region is sufficiently similar in sequence in both HIV-1 and HIV-2 for a single (common) oligonucleotide to be effective in inhibiting replication and gene expression of both viruses.

INHIBITION OF OTHER VIRUSES

The oligonucleotides and methods of the present invention can be used to inhibit the activity of viruses pathogenic to humans, animals or plants. As described above for inhibition of retroviruses, oligonucleotides complementary to viral RNA or viral mRNA for both DNA and RNA viruses would be used to inhibit viral replication and gene expression. It is also possible that in some cases, viral DNA will be the target of complementary oligonucleotides. Particularly useful target sites on the RNA are those which serve as binding or recognition sites for proteins (e.g., splice sites, protein initiation sites, polyadenylation signals, capped ends of mRNA, packaging signals, primer binding sites). In those instances in which the virus infects cells with a high ribonuclease H concentration, any sequence which is present within the viral RNA or mRNA and is not found in host RNA would be an acceptable site. Binding of the oligonucleotide to that site would result in enzymatic destruction of the hybridized RNA and hence inactivation of the virus.

Examples of target sites in other viruses are as follows:

1. Foot and Mouth Disease Virus
    1. 5'-CGT GAA TTC CTA CTT TCC TG.
    2. 5'-ACC CTA GCG CCC CCT TTC AA.
    The sequence of oligonucleotide 1 is complementary to the only known protein initiation site used by this virus. Robertson, B.H., et al., Journal of Virology, 54:651 (1985). The sequence of oligonucleotide 2 is complementary to the capped end of the virus. Harris, T.J.R., et al., Journal of Virology, 36:659 (1980).

2. Yellow Fever Virus
    1. 5'-CGA CCA GAC ATG TTC TGG TC.
    2. 5'-ATT AGC ACA CAG GAT TTA CT.
    The sequence of oligonucleotide 1 is complementary to the protein initiation site and that of oligonucleotide 2 is complementary to the capped end. Rice, C.M., et al., Science, 229:726 (1985).

### 3. Varicella-Zoster Virus

Varicella-Zoster virus has seventy genes and is replete with target sites, including splice sites. For example, the sequence 5'-CCT AGG CGT TAC AGG TCC CA, which is complementary to the protein initiation site in the first gene, can be used according to the method of the present invention to inhibit viral function. Davison, A.J. and J.E. Scott, Journal of General Virology, 67:1759 (1986).

### 4. Herpes Simplex Viruses Types 1 and 2

Like Varicella-Zoster virus, Herpes Simplex Viruses are extremely large viruses which have many target sites. A sequence complementary to the protein initiation site of the major capsid protein of Herpes Simplex 1 is: 5'-GGA GCG GCC ATG GGG TCG CG. Davison, A.J. and J.E. Scott, Journal of General Virology, 67:2279 (1986).

### 5. Plant Viruses

An example of a plant virus to which the hybridization arrest technique can be applied is cucumber mosaic virus. Its associated CARNA 5 satellite RNA has been sequenced, and this small RNA molecule has been shown to play a key role in inducing lethal necrosis in the plant. Richards, K.E. et al., Virology, 89:395 (1978). Sequence variation of this molecule from one strain to another is very limited. Thus, blockade of the function of this constant feature of the virus by hybridon therapy, administered by aerosol or other means, may provide a new form of plant viral chemotherapy.

### 6. Viroids

These covalently-closed, circular, single strand RNAs of low molecular weight (MW approximately 100,000) are perhaps the smallest known agents of infectious diseases in plants and animals. Diener, T.O., Viroids and Viroid Diseases, Wiley, New York (1979). Splice junctions of viroids with plant small nuclear RNAs, for example, offer an attractive target site for hybridon inhibition. Diener, T.O., Proceedings of the National Academy of Sciences, U.S.A., 78:5014 (1981). For example, aerosol therapy or addition to the soil in the region of plant roots might be therapeutically useful.

## INHIBITION OF OTHER INFECTIOUS AGENTS

The hybridization arrest method of the present invention is not limited to viruses. Other infectious agents, including bacteria, fungi, protozoa and worms may be inhibited. In these applications of the method, oligonucleotides which are complementary to RNA of the infectious agent which is essential to the viability of the infectious agent is used. The oligonucleotide is directed against the same type of target sites as described above and should not be complementary to host RNA sequences.

Examples of oligonucleotides inhibitory to the trypanosome Trypanosoma brucei gambiense (the protozoan responsible for African sleeping sickness) are:

1. 5'-TAC CAA TAT AGT ACA GAA AC
2. 5'-ACT GTT CTA ATA ATA GCG TT

Oligomer 1 is complementary to the splice donor site used to attach the mini-exon found at the 5'-end of all mRNA's in the organism. Oligomer 2 is complementary to the 5'-end of the same mini-exon. Campbell, D.A. et al., Nature, 311:350 (1984).

A similar situation is found in Leishmania. In this instance, there is considerable sequence homology between the protozoa (i.e., T. brucei and Leishmania). The oligomers complementary to Leishmania enriettii corresponding to those against T. brucei given above are:

1. 5'-TAC CAA TAA AGT ACA GAA AC
2. 5'-ACT GAT ACT TAT ATA GCG TT

An oligonucleotide complementary to the initiation site of a protein common in the female genital complex of the trematode Fasciola hepatica, a liver fluke, is 5'-TGA AAC TTC ATT TTT CAG TG. Zurita, M. et al., Proceedings of the National Academy of Sciences, USA, 84:2340 (1987).

Other target enzymes are chitin synthetase for anti-fungals and alanine racemase for antibacterials.

## USE OF HYBRIDONS FOR THE CONTROL OF NON-INFECTIOUS CONDITIONS

Hybridons may be of benefit in situations where it is desirable to reduce the level or activity of a given protein or enzyme. This is achieved using oligonucleotides complementary to unique sites on the mRNA for the protein or enzyme and using the same criteria for those sites described previously. Examples where this approach will be beneficial are given below.

### Contraceptives

Oligodeoxynucleotides that inhibit the synthesis of structural proteins or enzymes involved largely or exclusively in spermatogenisis, sperm motility, the binding of the sperm to the egg or any other step affecting sperm viability may be used as contraceptives for men. Similarly, contraceptives for women may be oligodeoxynucleotides that inhibit proteins or enzymes involved in ovulation, fertilization, implantation or in the biosynthesis of hormones involved in those processes.

### Cardiovascular Disorders

Hypertension can be controlled by oligodeoxynucleotides that suppress the synthesis of angiotensin converting enzyme or related enzymes in the renin/angiotensin system; platelet aggregation can be controlled by suppression of the synthesis of enzymes necessary for the synthesis of thromboxane A2 for use in myocardial and cerebral circulatory disorders, infarcts, arteriosclerosis, embolism and thrombosis; deposition of cholesterol in arterial wall can be inhibited by suppression of the synthesis of fattyacyl co-enzyme A: cholesterol acyl transferase in arteriosclerosis; inhibition of the synthesis of cholinephosphotransferase may be useful in hypolipidemia.

### Neural Disorders

There are numerous neural disorders in which hybridization arrest can be used to reduce or eliminate adverse effects of the disorder. For example, suppression of the synthesis of monoamine oxidase can be used in Parkinson's disease; suppression of catechol o-methyl transferase can be used to treat depression; and suppression of indole N-methyl transferase can be used in treating schizophrenia.

### Prostaglandins

Suppression of selected enzymes in the arachidonic acid cascade which leads to prostaglandins and leukotrienes may be useful in the control of platelet aggregation, allergy, inflammation, pain and asthma.

### Cancer

Suppression of the protein expressed by the multidrug resistance (mdr) gene, which is responsible for development of resistance to a variety of anti-cancer drugs and is a major impediment in chemotherapy may prove to be beneficial in the treatment of cancer. A sequence complementary to the protein initiation site in the closely-related mouse protein is 5'-CCC AGA ATC ATG CAC AGC TT. Gros, P. et al., Cell, 47:371 (1986). An oligonucleotide having this sequence or a sequence complementary to the human mdr gene, once elucidated, can be used to inhibit or suppress protein expression.

Suppression of proteins encoded by oncogenes, using the hybridization arrest method of the present invention and oligonucleotides of the proper sequence, may also prove useful.

### Others

There are other, varied disease states in which use of hybridization arrest may prove valuable. For example, suppression of the synthesis of aldose reductase may be of benefit in preventing complications in diabetes; suppression of glycolic acid oxidase may be of benefit to prevent crystallization of calcium oxalate (renal lithiation, primary hyperoxalurias, kidney stones); suppression of carbonic anhydrase may be useful in glaucoma, and suppression of hemoglobin synthesis in polycythemia vera might be therapeutically helpful.

The present invention will now be further illustrated by the following examples, which are not intended to be limiting in any way.

### EXAMPLE 1  Synthesis and Characterization of Oligodeoxynucleotides

Unmodified oligodeoxynucleotides were synthesized on an automated DNA synthesizer (Biosearch SAM I), using either standard triester or phosphoramidite chemistry. Gait, M.J. (Ed.), Oligonucleotide Synthesis, I.R.L. Press (1984). After deblocking, the products were purified first on Merck silica gel 60 thin layer chromatographic plates in i-propanol: concentrated ammonia water (55:35:10) and eluted with ethanol:water (1:3). Where necessary, further purification was performed by high pressure liquid chromatography, using a Waters SAX Radial-PAK catridge or by polyacrylamide gel electrophoresis (PAGE). The synthetic, preparative and analytical procedures have been described in detail. See Gait, M.J., above. The oligonucleotide with terminal 3'-deoxythymidine (ddT) was made by the solution phase triester method. This method is described in detail by Narang, S.A. et al., In: Methods in Enzymology, L. Grossman and K. Moldave (Ed.), 65:610-620, Academic Press (1980), the teachings of which are incorporated herein by reference. ddT (Sigma) was used directly in the coupling reaction without protecting groups. The final product was purified first on 2mm thick silica gel plates (Analtech) as above and subsequently by column chromatography on DEAE cellulose in a gradient of 0.02-0.8M triethylammonium bicarbonate.

Oligonucleotides were 5'-end-labeled by $T_4$ polynucleotide kinase, purified by polyacrylamide gel electrophoresis (PAGE) and sequenced by either the Maxam-Gilbert or wandering spot methods Maxam, A.M. and W. Gilbert, In: Methods in Enzymology, L. Grossman and K. Moldave (ed.) pp 499-560, Academic Press (1980); Jay, E. et al., Nucleic Acids Research, 1:331-353 (1974). For Maxam-Gilbert sequencing of fragments of this size, it was found necessary to increase reaction times up to 30 minutes at 37°. The presence of ddT at the 3' end of oligodeoxynucleotide did not seem to hinder the action of the exonuclease snake venom phosphodiesterase.

EXAMPLE 2 Oligodeoxynucleotide uptake studies

HeLa cells were grown in suspension culture, concentrated by centrifugation at 600 x g for 5 min. and resuspended at a concentration of 5 x $10^7$ to 5 x $10^8$ cells/ml of Dulbecco's modified Eagle's medium (DME) without serum and kept on ice. Synthetic oligodeoxynucleotides to be tested (10-30 nucleotides in length), were labeled with $^{32}$P at the 5'-end by polynucleotide kinase at 2 x $10^5$ cpm/nmol, dissolved in DME without serum, and added to the HeLa cell suspension (40 ul oligodeoxynucleotide solution to 0.7 ml ice cold HeLa cell suspension.). Alternatively, to generate an internally labelled oligonucleotide, two decamers, one of them 5'$^{32}$P labelled, were joined by T4 DNA ligase in the presence of an oligodeoxynucleotide (12 nucleotides long) part of which was complementary to the 5' end of one of the decamers and part of which was complementary to the 3' end of the other decamer. The concentration of labelled oligodeoxynucleotide in the HeLa cell suspension was usually 1 x $10^{-5}$ to 1 x $10^{-7}$M. Cells were incubated under sterile conditions at 37° for up to 20 hours. Samples were cooled at 0°, diluted to 10 ml with DME, and centrifuged lightly to pellet the cells. The supernatant fluid was poured off and saved and the centrifugation tube drained on filter paper. The cell pellets were then washed six times, each time in 9 ml of ice-cold DME. The supernatants were saved and monitored for $^{32}$P radioactivity. By the sixth wash, virtually no radioactivity was detected in the wash fluid. The cell pellets were then resuspended on 0.7 ml of ice cold DME and transferred to an electroporation cell. Electroporation was carried out at 0° by a variation of the technique described by Potter and co-workers in Potter et al., Proceedings of the National Academy of Sciences, USA, 81:7161-7165 (1984), the teachings of which are incorporated herein by reference. During electroporation, a short high voltage pulse was applied across the electroporation cuvette containing the cell pellets; in this way, the cell membranes were made temporarily leaky or porous, allowing oligonucleotides to pass out of the cells. The electroporation cuvette was kept in an ice bath for 15 min. following electroporation. The contents were transferred to a 1.5 ml Eppendorf microfuge tube, and centrifuged 5 min. at 12,000 x g. The supernatant solution (i.e., oligomer which has entered the cell) was removed, and radioactivity of both the supernatant and the pellet (which contained the nuclear and cell membrane component) was determined by scintillation counting.

Two other variants of this method were also used to determine whether externally added labeled oligodeoxynucleotides enter CEF and HeLa cells. In the case of CEF cells, which had been grown in monolayers in 75cm$^2$ Falcon flasks, the DME medium containing serum was removed, the cells were washed once with serum-lacking DME; 2 ml of DME containing $^{32}$P-labeled oligodeoxynucleotide were added; and the resulting combination was incubated at 37°C for 15 minutes. The cells were next washed six times at 37°C (ambient), each time with 10 ml of DME. 2 ml of 1N formic acid was then added, and the cells were kept on ice for 15 min. The same procedure was carried out with CEF or HeLa cells except that instead of 1N formic acid, distilled water was added after incubation to lyse the cells. Results were similar with both procedures; approximately half as much radioactivity was associated with the nuclear and cell membrane fraction (sedimented by centrifugation 5 min. at 12,000 x g) as was associated with the non-sedimenting fraction of the cell.

The possibility that treatment of labeled cells with either 1N formic acid or distilled water caused dissociation of radiolabeled oligomer (which had never entered the interior of the cell) from the cell membrane fraction was tested by using the above modified electroporation technique as described. Results using electroporation agree with those where cells were ruptured by hypotonicity or 1N formic acid.

These tests made it possible to assess uptake of $^{32}$P-oligonucleotides by the cultured cells described. Inhibition of viral replication by exogenous oligodeoxynucleotides depends upon their uptake in sufficient amounts by the cells; this is not the case when endogenously transcribed or microinjected anti-sense RNAs are used. The permeability of cultured mammalian cells to the oligodeoxynucleotides has been demonstrated by these tests to be as follows:

1) Under the experimental methods described, cellular uptake of 20-nucleotide sequences, labeled with $^{32}$P either internally or terminally, increased during the initial few hours of incubation. At an external concentration of $1 \times 10^{-7}$ M, after 4 hours of incubation at 37°C, the internally labeled 20-nucleotide sequence TAGTCTCAAT-$^{32}$P-GGGCTGATAA reached a concentration inside the HeLa cell of approximately $2 \times 10^{-9}$ M. In another experiment conducted using the same conditions described, at an external concentration of $2 \times 10^{-5}$ M, after 15 minutes of incubation at 37°C, the internally labelled 20-nucleotide sequence TAGTCTCAAT-$^{32}$P-GGGCTGATAA reached an apparent concentration inside the CEF cell of about $1.5 \times 10^{-6}$ M.

2) At 15 min. and 4 hour time periods labeled oligodeoxynucleotides released from chick embryo fibroblast cells by electroporation were largely intact, as judged both by migration on thin layer DEAE plates, in homomix V, Jay, E. et al., Nucleic Acids Research, 1:331-353 (1974), and by PAGE using oligodeoxynucleotide markers. However, degradation of oligodeoxynucleotides increased with incubation time. By 20 hours, a large fraction of oligodeoxynucleotide was degraded intracellularly and extracellularly, but undergraded oligomer was still detected, and thus endured long enough to have the desired inhibiting effect.

3) Terminally labeled oligodeoxynucleotides disappeared more rapidly than those labeled internally. This indicates that phosphomonoesterase activity is more rapid than endonuclease activity. This demonstrates that partially or completely ionic oligomers enter cells. Their location within the cells depends on the nature of the modifying groups on the oligonucleotides. Compelling evidence of cell entry also comes from the employment of oligomers covalently attached to the fluorescent dye rhodamine. These studies have been carried out with the collaboration of Dr. David Wolf of the Worcester Foundation for Experimental Biology. Rhodamine attached covalently to a nucleoside does not enter HeLa cells. Rhodamine attached to a 12-mer deoxynucleotide oligomer does not enter cells at zero time. Following 45 minutes incubation at 37°, rhodamine attached to the oligomer is visualized by intense fluorescence induced by the rhodamine exciting wavelength, and is found in the nucleoli and nuclear membrane of virtually all of the HeLa cells. Immediately following incubation at 37°, the cells had been washed free of rhodamine-oligomer in the incubation medium, and fluorescence was not seen on the cell surface.

EXAMPLE 3 Inhibition of HTLV-III replication by complementary oligodeoxynucleotides

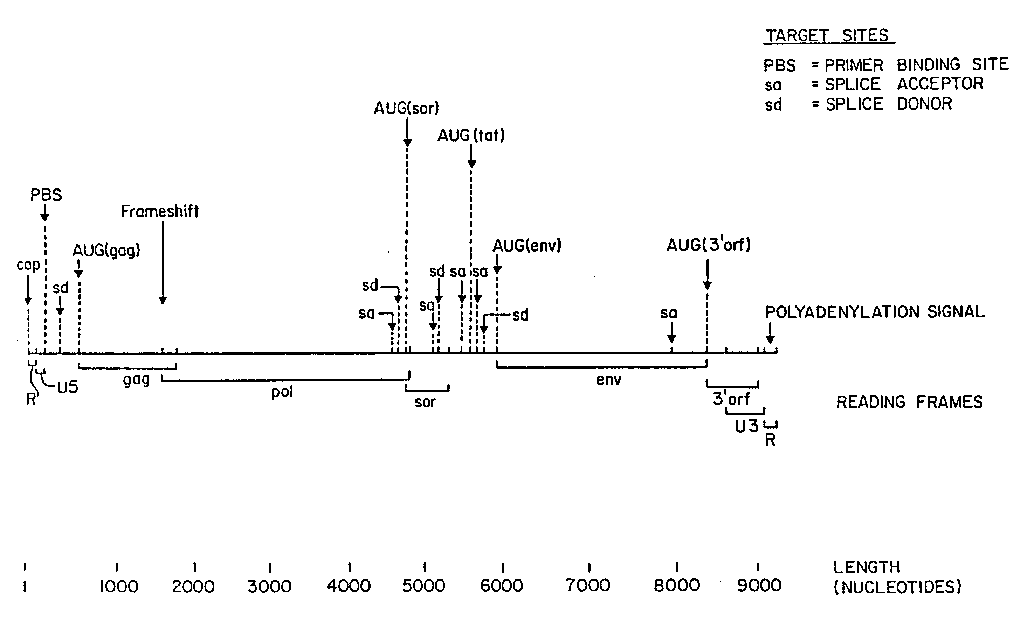

The primary nucleotide sequence of the HTLV-III/LAV genome has been determined during the past year by several groups of investigators, as indicated above. The following regions of the genome were selected as oligonucleotide competitive inhibition targets: a) a sequence of nucleotides 5' to the primer tRNA[lys] binding(association) site; b) a sequence straddling the primer binding site and the adjacent region, in the 5' direction; c) a sequence at the primer binding site and d) sequences from the splice sites (i.e., splice donor site, splice acceptor site) of the pre-mRNA that expresses the 3'-open reading frame regions. Sodroski, J. et al., Journal of Virology, 55:831-835 (1985); Wong-Staal, F. and Gallo, R.C., Nature, 317:395-403 (1985). Their locations on the HTLV-III/LAV genome are indicated in Figure 1.

A. Sequences complementary to the primer binding site and sequences vicinal to the primer binding site in a 5' direction

Several sequences complementary to regions immediately adjacent, in a 5'-direction, to the tRNA[lys] primer binding site in HTLV-III, or complementary to the primer binding site were synthesized. These are a 12-nucleotide sequence (5'CTGCTAGAGATddT) a 20-nucleotide sequence (5'-CTGCTAGAGATTTTCCACAC), and a 26-nucleotide sequence with a 3' terminal non-complementary tail of $(pA)_3$ (5'-TTCAAGTCCCT-GTTCGGGCGCCAAAA). As shown in Table 1, the 12-nucleotide sequence is complementary to a sequence of nucleotides close to the primer binding site (in the 5' direction); the 20-nucleotide sequence is also complementary to a sequence close to the primer binding site in the 5' direction, and includes the first 11 nucleotides of the 12-nucleotide sequence, as well as nine additional nucleotides. The 26-nucleotide sequence is complementary to the primer binding site. These oligodeoxynucleotides were tested on cultures of HTLV-III-infected cells; they were added to the cultures at the concentrations shown in Table 1 (column 3). Both reverse transcriptase activity and production of viral-encoded p15 and p24 proteins were measured to determine inhibition of viral replication and inhibition of gene expression, respectively.

Table I: Inhibition of HTLV-III Replication and Protein Expression by Complementary Oligodeoxynucleotides

| Sequence | Oligomer Length | Conc. µg/ml | HTLV-III Binding Site | Cell Line | HTLV-III Added | Percent Inhibition RT | Percent Inhibition p15 | Percent Inhibition p24 |
|---|---|---|---|---|---|---|---|---|
| CCCCAACTGTGTACT | 15 | 5 | none | 119 | - | 0 | 0 | 0 |
| " | " | 10 | none | 119 | + | 0 | 0 | 0 |
| CTGCTAGAGATddT | 12 | 5 | 5'-vicinal to PBS | PB | + | 30 | 0 | 17 |
| " | " | 10 | " | " | + | 36 | 0 | 50 |
| " | " | 20 | " | " | + | 40 | 35 | 36 |
| " | " | 5 | " | 119 | + | 10 | 15 | 35 |
| " | " | 10 | " | " | + | 17 | 15 | 50 |
| " | " | 10 | " | 119 | + | 0 | 10 | 12 |
| " | " | 20 | " | " | + | 0 | 28 | 38 |
| CTGCTAGAGATTTTCCACAC | 20 | 50 | " | PB | + | 50 | 50 | 50 |
| " | " | 10x3+ | " | 119 | + | 50 | 75 | 75 |
| " | " | 50 | " | " | + | 23 | 27 | 30 |
| TTCAAGTCCCTGTTC-GGGCGCCAAA | 26 | 50 | at PBS‡ | 119 | + | 80 | 4 | 8 |
| GCGTACTCACCAGTCGCCGC | 20 | 50 | splice donor site | 119 | + | 85 | 40 | 60 |
| CTGCTAGAGATTAA | 14 | 50 | 5'-vicinal to PBS § | 119 | + | 75 | 8 | 11 |
| ACACCCAATTCTGAAAATGG | 20 | 50 | splice acceptor site | 119 | + | 67 | 95 | 88 |

PB: peripheral human blood cells

119: transformed immortalized human T cell line

+ 10 ug/ml. on days 1, 2 and 3 from time of infection

‡ Has 3 non-complementary bases at 3' end

§ Has 2 non-complementary bases at 3' end

Λ+ PBS indicates directly competing at primer binding site

Reverse transcriptase activity was measured by the method described by Sarin and co-workers, which is a modification of an earlier method described by Baltimore and Smoller. The modified method is described in Sarin, P.S. et al., Biochimica Biophysica Acta, 470:198-206 (1977) and the earlier method in Baltimore, D. and Smoller, D., Proceedings of the National Academy of Sciences, U.S.A., 68: 1507-1511 (1971); the teachings of both references are incorporated herein by reference.

HTLV-III-protein expression was measured by immunofluorescence using monoclonal antibodies to HTLV-III p15 and p24 as described in Sarin et al., Biochemistry and Pharmacology, 34:4075-4078 (1985), the teachings of which are incorporated herein by reference.

13

In separate experiments, peripheral human blood cells and transformed T-lymphocyte (H9) cells were infected with HTLV-III; the oligodeoxynucleotides were added just once (at time zero), unless otherwise indicated. Assays for inhibition were carried out at 96 hours.

B. Sequences complementary to splice sites of pre-mRNA

A 20-nucleotide sequence complementary to a splice donor site from the 3'-open reading frame region, and a 20-nucleotide sequence complementary to a splice acceptor site were produced. These oligodeoxynucleotides were tested as described in part A (above); their effects were also measured through determintion of reverse transcriptase activity and production of viral-encoded proteins.

C. Results of inhibition tests

The results of testing using the oligodeoxynucleotides described in A) and B) of this example are shown in Table 1. The greatest inhibition occurred when an oligonucleotide having the sequence ACACCCAATTCTGAAAATGG, which is complementary to the splice acceptor site in H9 cells, was added at 50 ug/ml ($9 \times 10^{-6}$ M). Percent inhibition as shown in the table is based on comparison with control values obtained for HTLV-III-infected cells incubated without oligodeoxynucleotide. As indicated in Table 1 (columns 7-9), reverse transcriptase activity was inhibited by 67%, p15 protein production by 95% and p27 protein production by 88% when this sequence was used. The oligodeoxynucleotide was given just once (at time zero), and inhibitory effects were observed at 96 hours. Marked inhibition was also found with the other oligodeoxynucleotides, as shown in Table 1.

For example, when the 12-mer sequence complementary to the region of the HTLV-III/LAV genome adjacent, in the 5' direction, to the tRNA$^{lys}$ primer binding site was added to HTLV-III-infected cells at the concentrations shown in Table 1, reverse transcriptase activity was inhibited from 10-17% in H9 cells and 30-40% in PB cells. Viral p15 and p24 protein production was inhibited by 15% and by 35-50%, respectively, in H9 cells; in PB cells, inhibition of p15 protein production ranged from 0-35% and of p24 protein production, from 17-50%. When the 20-nucleotide sequence was used, reverse transcriptase activity was inhibited in H9 cells by 23-50% and viral protein production by 27-75%. Fifty percent inhibition of all three activities was observed in PB cells as a result of addition of the 20-nucleotide sequence. Based on work on inhibition of Rous sarcoma virus in tissue culture, it seems likely that daily addition of competitor oligodeoxynucleotide is more effective than a single dose at time zero. Zamecnik, P.C. and M.L. Stephenson, Proceedings of the National Academy of Sciences, USA, 75: 280-284 (1978). This is also consistent with time-related intracellular and extracellular degradation of added oligodeoxynucleotide, since measurement of efficacy occurs at 96 hours. Although overall variation in assays of other chemotherapeutic agents for HTLV-III is in the vicinity of ± 5 percent, it is considerably higher where oligodeoxynucleotides are being tested (cf. Table I). This may be related to variable nuclease activity, both intracellular and extracellular, in tissue cultures of H9 and PBS cells. Such an effect would be more marked at lower concentration of oligodeoxynucleotides. Oligodeoxynucleotides blocked at the 3' end by ddT, the isourea group, other chain terminators or by internucleoside phosphate modifications, such as phosphorothioates, phosphoromorpholidates, phosphoropiperazines, and phosphoroamidates in general may prove to be more effective inhibitors than those described above. For example, work on inhibition of Rous sarcoma virus has shown that 5' and the 3' end blocked hybridon was a more effective inhibitor than a hybridon having an unblocked 3' and 5' end. Proceedings of the National Academy of Sciences, U.S.A., 75:280-284 (1978). This is particularly pertinent to prevention of initiation of replication at loci close to the primer binding site.

EXAMPLE 4 The Use of Oligodeoxynucleotides for the inhibition of growth and expression of HIV in tissue culture

Growth and expression of HIV in suspension tissue cutures of H9 cells have been monitored by assay of viral reverse transcriptase, p17 and p24 viral protein synthesis, syncytial formation, and growth of host cells. Virus and oligodeoxynucleotide oligomers were added to the cultures at zero time, and incubation was carried out for 96 hours at 37°C. A variety of oligomers of the same sequence (either 15 mers or 20 mers) have been compared for inhibitory properties; oligomers compared included unmodified oligomers; oligomers with all internucleoside phosphates modified as phosphorothioates, methylphosphonates, phosphoromorpholidates or phosphoropiperazine derivatives; and oligomers with replacement of just 3' and 5' terminal and penultimate internucleoside phosphates by the above-mentioned modifications.

Target sites for potential inhibition were also varied, with principal focus on the splice donor and acceptor sitse, plus the initiation and cap sites. Target sites and results are shown in Tables 3-7. Within a single experiment, comparisons of degrees of inhibition of virus growth by these modified and unmodified oligomers are reproducible. Considerable variability in the degree of inhibition induced by the same oligomer in experiments repeated at different times and with different batches of virus occurred. This variation is unexplained.

Inhibition of as great as 80-95% of the abovementioned parameters of growth and expression of HIV was observed with initial concentrations of 10-100 ug per ml of all these classes of oligomers. Phosphorothioates consisting of homopolymer G's, C's, and T's and a random sequence 20-mer phosphorothioate also gave good inhibition of virus growth. It is possible that more than one mechanism of viral inhibition by oligodeoxynucleotides operated in these in vitro studies.

## TABLE 2

### THE EFFECT OF UNMODIFIED OLIGONUCLEOTIDES (20-mers) ON HIV REPLICATION IN H9 CELLS

| Sequence 5' ——→ 3' | Binding[1] Site | Function[2] | %Inhibition[3] Syncytia | | | p24 | | | Toxicity[4] 100ug/ml |
|---|---|---|---|---|---|---|---|---|---|
| | | | 4 | 20 | 100 | 4 | 20 | 100 | |
| CTG GTC TAA CCA GAG AGA CC | 1-20 | Cap | 30 | 54 | 71 | 48 | 72 | 72 | 0 |
| TGA GGC TTA TGC AGT GGG TT | 54-73 | 5'untranslated | 34 | 64 | 87 | | | | |
| CTG CTA GAG ATT TTC CAC AC | 162-181 | Next to P.B.S. | 39 | 50 | 76 | 60 | 64 | 44 | 0 |
| AAG TCC CTG TTC GGG CGC CA | 182-201 | P.B.S | 29 | 56 | 56 | 64 | 60 | 60 | 0 |
| GCG TAC TCA CCA GTC GCC GC | 280-299 | Splice Donor | 35 | 42 | 47 | 32 | 68 | 68 | 0 |
| CTC GCA CCC ATC TCT CTC CT | 327-346 | gag initiator | 21 | 9 | 45 | 0 | 24 | 60 | 18 |
| TCT TCC CTA AAA AAT TAG CC | 1625-1644 | frame shift | 9 | 48 | 44 | 4 | 28 | 44 | 0 |
| TCT GCT GTC CCT GTA ATA AA | 4484-4503 | splice acceptor | 1 | 24 | 56 | 0 | 52 | 80 | 10 |
| GCC CCT TCA CCT TTC CAG AG | 4534-4553 | splice donor | 21 | 33 | 56 | 0 | 20 | 26 | 8 |
| CTG TTT TCC ATA ATC CCT AA | 4613-4632 | sor initiator | 15 | 65 | 69 | 33 | 60 | 53 | 0 |
| ATA GCA GAG TCT GAA AAA CA | 4961-4980 | splice acceptor | 52 | 56 | 59 | 0 | 40 | 40 | 0 |
| GAG ATC CTA CCT TGT TAT GT | 5035-5054 | splice donor | 27 | 60 | 46 | 20 | 46 | 13 | 0 |
| ACA CCC AAT TCT GAA AAT GG | 5349-5368 | splice acceptor | 39 | 56 | 73 | 40 | 60 | 60 | 0 |
| ACT GGC TCC ATT TCT TGC TC | 5403-5422 | tat initiator | 48 | 61 | 42 | 0 | 0 | 0 | 8 |
| CCG CTT CTT CCT GCC ATA GG | 5548-5567 | splice acceptor art initiator | 27 | 41 | 54 | 47 | 20 | 33 | 18 |
| TAC TAC TTA CTG CTT TGA TA | 5617-5636 | splice donor | 25 | 21 | 44 | 0 | 26 | 0 | 10 |
| TTC ACT CTC ATT GCC ACT GT | 5796-5613 | env initiator | 50 | 44 | 36 | 60 | 0 | 0 | 14 |
| GGA GGT GGG TCT GAA ACG AT | 7947-7966 | splice acceptor | 52 | 76 | 73 | 20 | 40 | 53 | 0 |
| TTG CCA CCC ATC TTA TAG CA | 8366-8385 | 3'-orf initiator | 41 | 61 | 56 | 33 | 33 | 33 | 18 |
| GGC AAG CTT TAT TGA GGC TT | 9183-9202 | polyadenylation signal | 37 | 71 | 85 | 53 | 53 | 67 | 20 |
| CAG TCA GTL AGT CAG TCA GT | N/A | non complementary control | 0 | 0 | 0 | 0 | 7 | 0 | 33 |

## TABLE 2 (cont'd)

COMPARISON OF DIFFERENT CHAIN LENGTHS

| | | | | | | | | |
|---|---|---|---|---|---|---|---|---|
| ACA CCC AAT TCT GAA AAT GG | 5349-5368 | splice acceptor | 39 | 56 | 73 | 40 | 60 | 60 |
| ACC CAA TTC TGA AAA T | 5351-5366 | splice acceptor | 55 | 55 | 64 | | | |
| CCA ATT CTG AAA | 5352-5364 | splice acceptor | 9 | 55 | 64 | | | |

Footnotes

[1] Numbering is that used by M.A. Muesing et al., Nature 313:450-458 (1985).
[2] Function of site to which hybridon binds P.B.S. = primer binding site.
[3] % inhibition of syncytia formation and p24 expression is reported using concentrations of oligonucleotide of 4, 20 and 100 ug/ml.
[4] Toxicity is the % reduction in the number of cells at 96 hours after exposure to 100 ug/ml of oligonucleotide compared to control cells. No virus was used in this determination.

## TABLE 3

INHIBITION OF HIV REPLICATION IN H9 CELLS BY OLIGONUCLEOTIDE PHOSPHOROTHIOATES[*]

| Sequence 5' ⟶ 3' | Binding[1] Site | Function[2] | Conc. ug/ml | %Inhibition Syncytia | p24 | Toxicity[3] 100 ug/ml |
|---|---|---|---|---|---|---|
| ACA CCC AAT TCT GAA AAT GG | 5349-5368 | Splice Acceptor | 100 | 100 | 100 | 0 |
| | | | 20 | 86 | 91 | |
| | | | 4 | 38 | 63 | |
| CCC AAT TCT GAA AAT | 5351-5365 | Splice Acceptor | 100 | 95 | 95 | 68 |
| | | | 20 | 0 | 54 | |
| | | | 4 | 0 | 9 | |
| CTA ACC AGA GAG ACC | 1-15 | Cap | 100 | 50 | 64 | 30 |
| | | | 20 | 25 | 29 | |
| | | | 4 | 0 | 72 | |
| CGT ACT CAC CAG TCG | 281-296 | Splice Donor | 100 | 64 | 63 | 0 |
| | | | 20 | 68 | 49 | |

[*] Internucleoside phosphate is replaced by $-O-\overset{\overset{O}{\|}}{\underset{\underset{S^{\ominus}}{\|}}{P}}-O-$

[1] Numbering is that used by M.A. Muesing et al., Nature, 313:450-458 (1985).
[2] Function of site to which hybridon binds P.B.S. = primer binding site.
[3] Toxicity is the % reduction in the number of cells at 96 hours after exposure to 100 ug/ml of oligonucleotide compared to control cells. No virus was used in this determination.

EP 0 402 402 B1

## TABLE 4

INHIBITION OF HIV REPLICATION IN H9 CELLS BY OLIGONUCLEOTIDE PHOSPHOROMORPHOLIDATES[*]

| Sequence 5' ⟶ 3' | Binding[1] Site | Function[2] | Conc. ug/ml | %Inhibition Syncytia | p24 | Toxicity[3] 100 ug/ml |
|---|---|---|---|---|---|---|
| CCC AAT TCT GAA AAT | 5351-5365 | Splice Acceptor | 500 | 90 | 84 | |
| | | | 100 | 49 | 75 | 14 |
| | | | 20 | 47 | 46 | |
| | | | 4 | 52 | 43 | |
| CTA ACC AGA GAG ACC | 1-15 | Cap | 500 | 86 | 66 | 25 |
| | | | 100 | 72 | 62 | |
| | | | 20 | 55 | 43 | |
| | | | 4 | 43 | 43 | |

[*] Internucleoside phosphate is replaced by

$$-O-\overset{\overset{\displaystyle O}{\|}}{\underset{\underset{\displaystyle N}{|}}{P}}-O-$$

[1] Numbering is that used by M.A. Muesing et al., Nature, 313:450-458 (1985).
[2] Function of site to which hybridon binds P.B.S. = primer binding site.
[3] Toxicity is the % reduction in the number of cells at 96 hours after exposure to 100 ug/ml of oligonucleotide compared to control cells. No virus was used in this determination.

## TABLE 5

INHIBITION OF HIV REPLICATION IN H9 CELLS BY AN OLIGONUCLEOTIDE PHOSPHOROBUTYLAMIDATE*

| Sequence 5' ⟶ 3' | Binding[1] Site | Function[2] | Conc. ug/ml | %Inhibition Syncytia | p24 | Toxicity[3] 100 ug/ml |
|---|---|---|---|---|---|---|
| CGT ACT CAC CAG TCG | 281-296 | Splice Donor | 500 | 83 | 69 | 14 |
| | | | 100 | 58 | 43 | |
| | | | 20 | 47 | 34 | |
| | | | 4 | 59 | 23 | |
| | | | 1 | 53 | 23 | |

\* Internucleoside phosphate is replaced by

$$-\text{O}-\overset{\displaystyle \overset{\text{O}}{\|}}{\underset{\displaystyle \underset{\text{CH}_3}{\underset{|}{\underset{\text{CH}_2}{\underset{|}{\underset{\text{CH}_2}{\underset{|}{\underset{\text{CH}_2}{\underset{|}{\text{N}}}}}}}}}{\text{P}}}-\text{O}-$$

[1] Numbering is that used by M.A. Muesing et al., Nature, 313:450-458 (1985).
[2] Function of site to which hybridon binds P.B.S. = primer binding site.
[3] Toxicity is the % reduction in the number of cells at 96 hours after exposure to 100 ug/ml of oligonucleotide compared to control cells. No virus was used in this determination.

EP 0 402 402 B1

TABLE 6

INHIBITION OF HIV REPLICATION IN H9 CELLS BY OLIGONUCLEOTIDES CONTAINING SOME METHYLPHOSPHONATES[*]

| Sequence 5'——→ 3' | Binding[1] Site | Function[2] | Conc. ug/ml | %Inhibition Syncytia | p24 | Toxicity[3] 100 ug/ml |
|---|---|---|---|---|---|---|
| ApApT TCT GpAA AAT GGA TApАp A | 5355-5374 | Splice Acceptor | 500 | 55 | 39 | 20 |
| | | | 100 | 51 | 34 | |
| | | | 20 | 19 | 55 | |
| | | | 4 | 10 | 0 | |
| CpCp AAT TCT GAA ATT GCA TApАp A | 5359-5374 | Splice Acceptor | 500 | 57 | 44 | 25 |
| | | | 100 | 65 | 22 | |
| | | | 20 | 29 | 33 | |
| | | | 4 | 24 | 36 | |
| ACpAp CpCpCp ApApTp TpCpTp GpApAp | | | 20 | 75 | 99 | |
| ApApTp GpG | | | 0-8 | 39 | 67 | |

* $p = -o-\overset{\overset{O}{\|}}{\underset{\underset{CH_3}{|}}{P}}-o-o$    Other internucleoside linkages are normal.

[1] Numbering is that used by M.A. Muesing et al., Nature, 313:450-458 (1985).
[2] Function of site to which hybridon binds P.B.S. = primer binding site.
[3] Toxicity is the % reduction in the number of cells at 96 hours after exposure to 100 ug/ml of oligonucleotide compared to control cells. No virus was used in this determination.

TABLE 7

INHIBITION OF HIV REPLICATION BY OLIGONUCLEOTIDES WITH 5'-MODIFICATIONS

| Sequence 5' $\longrightarrow$ 3' | Binding[1] Site | Function[2] | Conc. ug/ml | %Inhibition Syncytia | %Inhibition p24 | Toxicity[3] 100 ug/ml |
|---|---|---|---|---|---|---|
| R-CA CCC AAT TCT GAA AAT GGA | 5350-5369 | Splice Acceptor | | | | |
| R = Acridine-linker | | | 100 | 73 | 50 | 47 |
| | | | 20 | 64 | 17 | |
| | | | 4 | 33 | 22 | |
| R = CH₃(CH₂)₂ | | | 500 | 87 | 61 | 57 |
| | | | 100 | 74 | 58 | |
| | | | 20 | 72 | 64 | |
| | | | 4 | 68 | 61 | |

INHIBITION OF HIV REPLICATION BY OLIGONUCLEOTIDES WITH 5'-MODIFICATIONS & METHYLPHOSPHONATES

| Sequence 5' $\longrightarrow$ 3' | Binding[1] Site | Function[2] | Conc. ug/ml | %Inhibition Syncytia | %Inhibition p24 | Toxicity[3] 100 ug/ml |
|---|---|---|---|---|---|---|
| R-CA CCC AAT TCT GAA AAT GpGpA | 5350-5369 | Splice Acceptor | | | | |
| R = Acridine-linker; p = internucleoside methylphosphonate | | | 100 | 74 | 69 | 74 |
| | | | 20 | 55 | 72 | |
| | | | 4 | 45 | 72 | |
| R = CH₃(CH₂)₉ $O-\overset{O}{\overset{\|}{P}}-O-$ $\underset{O^-}{\|}$  p = internucleoside methylphosphonate | | | 100 | 36 | 11 | 62 |
| | | | 20 | 26 | 22 | |
| | | | 4 | 33 | 44 | |

FOOTNOTES:

1 Numbering is that used by M.A. Muesing et al., Nature, 313:450-458 (1985).
2 Function of site to which hybridon binds P.B.S. = primer binding site.
3 Toxicity is the % reduction in the number of cells at 96 hours after exposure to 100 ug/ml of oligonucleotide compared to control cells. No virus was used in this determination.

Equivalents

Those skilled in the art will recognize, or be able to ascertain using no more than routine experimentation, many equivalents to the specific embodiments of the invention described specifically herein. Such equivalents are intended to be encompassed in the scope of the following claims.

22

**Claims**

1.  A preparation for use in therapy comprising a single stranded modified oligonucleotide of 8-50 nucleotides in length, wherein the modified oligonucleotide is one which is modified at an internucleoside phosphate, being a phosphomorpholidate, phosphopiperazine or phosphoromidate derivative, the oligonucleotide being capable of selectively hybridizing with a highly conserved region of the HTLV-III genome to induce selective hybridization arrest of HTLV-III replication and/or gene expression, the highly conserved region comprising any of:

    (i) the tRNA$^{lys}$ primer binding site;

    (ii) regions of the HTLV-III genome vicinal in the 5' direction to the tRNA$^{lys}$ primer binding site;

    (iii) the tRNA$^{lys}$ primer binding site and regions of the HTLV-III genome vicinal in the 5' direction to the tRNA$^{lys}$ primer binding site;

    (iv) the mRNA donor splice sites;

    (v) the mRNA acceptor splice sites;

    (vi) the initiator codon for the gag gene;

    (vii) the initiator codon for the env gene;

    (viii) the initiator codon for the tat gene;

    (ix) the initiator codon for the sor gene;

    (x) the initiator codon for the 3' orf gene;

    (xi) the cap nucleotide of the HTLV-III genome;

    (xii) the art gene or portions thereof;

    (xiii) the region of HTLV-III genome encoding a frameshift.

2.  A preparation according to claim 1, wherein the modified oligonucleotide is selected from the group consisting of:

    a) oligonucleotide phosphoromorpholidates represented in Table 4;

    b) the oligonucleotide phosphorobutylamidates represented in Table 5;

    c) oligonucleotides in which all internucleoside phosphates are modified as phosphoromorpholidates, or phosphoropiperazine derivatives, or a combination thereof;

    d) oligonucleotides in which, in addition to the modifications defined in claim 1, the 3' terminal and penultimate internucleoside phosphates, the 5' terminal and penultimate internucleoside phosphates or both the 3' and 5' terminal and penultimate internucleoside phosphates are modified as phosphorothioates, methylphosphonates, phosphoromorpholidates, phosphoropiperazine derivatives, or a combination thereof;

    e) oligonucleotides complementary to the HTLV-III primer binding site in which, in addition to the modifications defined in claim 1, the 3' end, sequences vicinal to the primer binding site in the 5' direction, alone or in combination, are blocked in such a manner that the region is unable to serve as a template for the reverse transcriptase activity of HTLV-III;

    f) oligonucleotides modified at the 5' end by addition of a lipophilic group, polylysine, polyarginine or another agent which enhances uptake of the oligonucleotides, in addition to the modifications defined in claim 1,

    g) oligonucleotides modified by additon of an intercalating agent, in addition to the modifications defined in claim 1,

    h) oligonucleotides comprising one or more chemically reactive groups capable of crosslinking the region or regions of the HTLV-III genome to which they are complementary, in addition to the modifications defined in claim 1,

    i) oligonucleotides comprising one or more chemically reactive groups capable of cleaving the region or regions of the HTLV-III genome to which are are complementary, in addition to the modifications defined in claim 1.

3.  An in vitro method of inhibiting HTLV-III replication, HTLV-III gene expression or both in cells containing HTLV-III, comprising introducing into the cells a modified oligodeoxynucleotide according to claim 1 or claim 2.

4.  Use of a preparation (or modified derivative thereof) according to any one of the preceding claims for the manufacture of a medicament for use in AIDS chemotherapy, e.g. in inhibiting HTLV-III replication and/or gene expression in peripheral human blood cells (e.g. human T lymphocytes) infected with HTLV-III.

**Patentansprüche**

1. Eine Zubereitung zur therapeutischen Anwendung umfassend ein einzelsträngiges modifiziertes Oligonucleotid von 8-50 Nucleotiden, in dem das modifizierte Oligonucleotid, das an einem Internucleosid-Phosphat modifiziert ist, ein Phosphormorpholidat, Phosphorpiperazin oder Phosphoramidat-Derivat ist, das Oligonucleotid fähig zur selektiven Hybridisierung mit einer hochkonservierten Region des HTLV-III Genoms ist, um die selektive Hybridisierung zu induzieren, die die HTLV-III Replikation und/oder Genexpression aufhält, wobei die hochkonservierte Region irgendeine der folgenden umfaßt:

(i) die tRNA$^{lys}$ Primerbindungsstelle,

(ii) Regionen des HTLV-III Genoms, benachbart in der 5'-Richtung zur tRNA$^{lys}$ Primerbindungsstelle,

(iii) die tRNA$^{lys}$ Primerbindungsstelle und Regionen des HTLV-III Genoms benachbart in der 5'-Richtung zur tRNA$^{lys}$ Primarbindungsstelle,

(iv) die mRNA Donor-Spleißstellen,

(v) die mRNA Akzeptor-Spleißstellen,

(vi) das Initiationskodon für das gag-Gen,

(vii) das Initiationskodon für das env-Gen,

(viii) das Initiationskodon für das tat-Gen,

(ix) das Initiationskodon für das sor-Gen,

(x) das Initiationskodon für das 3'-orf-Gen,

(xi) das cap-Nucleotid des HTLV-III Genoms,

(xii) das art-Gen oder Teile davon,

(xiii) die Region des HTLV-III Genoms, die ein Frameshift kodiert.

2. Eine Zubereitung nach Anspruch 1, in der das modifizierte Oligonucleotid ausgewählt ist aus der Gruppe bestehend aus:

a) Oligonucleotid-Phosphormorpholidate dargestellt in Tabelle 4;

b) die Oligonucleotid-Phosphorbutylamidate dargestellt in Tabelle 5;

c) Oligonucleotide, in denen alle Internucleosid-Phosphate modifiziert sind als Phosphormorpholidate oder Phosphorpiperazin-Derivate, oder eine Kombination davon;

d) Oligonucleotide, in denen zusätzlich zu den Modifikationen, definiert in Anspruch 1, das 3' terminale und vorletzte Internucleosid-Phosphat, das 5' terminale und vorletzte Internucleosid-Phosphat oder beide, das 3' und das 5' terminale und vorletzte Internukleosid-Phosphat modifiziert sind als Phosphorthioate, Methylphosphonate, Phosphormorpholidate, Phosphorpiperazin-Derivate oder eine Kombination davon;

e) Oligonucleotide komplementär zu der HTLV-III Primerbindungsstelle, in denen zusätzlich zu den Modifikationen, definiert in Anspruch 1, das 3'-Ende von Sequenzen benachbart zur Primarbindungsstelle in der 5'-Richtung alleine oder in Kombination in der Art blockiert sind, daß die Region unfähig ist, als ein Templat für die Reversetranscriptase-Aktivität von HTLV-III zu dienen;

f) Oligonucleotide modifiziert an dem 5'-Ende durch Hinzufügen einer lipophilen Gruppe, Polylysin, Polyarginin oder anderer Mittel, die die Aufnahme der Oligonucleotide erhöhen, zusätzlich zu den Modifikationen, definiert in Anspruch 1;

g) Oligonucleotide modifiziert durch Zugabe eines interkalierenden Mittels, zusätzlich zu den Modifikationen, definiert in Anspruch 1;

h) Oligonucleotide umfassend eine oder mehrere chemisch reaktive Gruppen, fähig zur Quervernetzung der Region oder Regionen des HTLV-III Genoms, zu dem sie komplementär sind, zusätzlich zu den Modifikationen, definiert in Anspruch 1;

i) Oligonucleotide umfassend eine oder mehrere chemisch reaktive Gruppen, fähig zur Spaltung der Region oder Regionen des HTLV-III Genoms, zu dem sie komplementär sind, zusätzlich zu den Modifikationen, definiert in Anspruch 1.

3. Ein in vitro Verfahren zur Inhibierung der HTLV-III Replikation der HTLV-III Genexpression oder von beiden in HTLV-III enthaltenden Zellen, umfassend das Einführen eines modifizierten Oligonucleotids nach Anspruch 1 oder 2 in Zellen.

4. Die Verwendung einer Zubereitung (oder eines modifizierten Derivats hiervon) nach einem der vorhergehenden Ansprüche zur Herstellung eines Medikaments zur Verwendung in der AIDS-Chemotherapie, z.B. durch Inhibierung der HTLV-III Replikation und/oder Genexpression in peripheren menschlichen Blutzellen (z.B. menschlichen T-Lymphozyten) infiziert mit HTLV-III.

**EP 0 402 402 B1**

**Revendications**

1. Préparation destinée à être utilisée en thérapeutique, comprenant un oligonucléotide modifié monocaténaire de 8 à 50 nucléotides de longueur, dans laquelle l'oligonucléotide modifié est un oligonucléotide qui est modifié au niveau d'un phosphate internucléosidique, consistant en un phosphomorpholidate, un dérivé de phosphopipérazine ou un phosphoro-amidate, l'oligonucléotide étant apte à l'hybridation sélective avec une région hautement conservée du génome de HTLV-III pour induire un arrêt, par hybridation sélective, de la réplication et/ou de l'expression de gènes de HTLV-III, la région hautement conservée comprenant l'un quelconque des éléments suivants :

(i) le site de liaison d'amorce d'ARNt$^{lys}$ ;

(ii) des régions du génome de HTLV-III vicinales, dans le sens 5', du site de liaison d'amorce d'ARNt$^{lys}$ ;

(iii) le site de liaison d'amorce d'ARNt$^{lys}$ et des régions du génome de HTLV-III vicinales, dans le sens 5', du site de liaison d'amorce d'ARNt$^{lys}$ ;

(iv) les sites donneurs d'épissage d'ARNm ;

(v) les sites accepteurs d'épissage d'ARNm ;

(vi) le codon initiateur pour le gène gag ;

(vii) le codon initiateur pour le gène env ;

(viii) le codon initiateur pour le gène tat ;

(ix) le codon initiateur pour le gène sor ;

(x) le codon initiateur pour le gène 3' orf ;

(xi) le nucléotide de coiffe du génome de HTLV-III ;

(xii) le gène art ou ses portions ;

(xiii) la région du génome de HTLV-III codant pour un décalage de cadre.

2. Préparation suivant la revendication 1, dans laquelle l'oligonucléotide modifié est choisi dans le groupe consistant en :

a) des oligonucléotide-phosphoromorpholidates représentés sur le tableau 4 ;

b) les oligonucléotide-phosphorobutylamidates représentés sur le tableau 5 ;

c) des oligonucléotides dans lesquels tous les phosphates internucléosidiques sont modifiés sous forme de phosphoromolidates, ou de dérivés de phosphoropipérazine ou bien d'une de leurs associations ;

d) des oligonucléotides dans lesquels, en plus des modifications définies dans la revendication 1, le dernier et l'avant-dernier internucléoside-phosphates 3', le dernier et l'avant-dernier internucléoside-phosphates 5' ou bien à la fois le dernier et l'avant-dernier internucléoside-phosphates 3' et le dernier et l'avant-dernier internucléoside-phosphates 5' sont modifiés sous forme de phosphorothioates, méthylphosphonates, phosphoromorpholidates, dérivés de phosphoropipérazine ou une de leurs associations ;

e) des oligonucléotides complémentaires du site de liaison d'amorce de HTLV-III, dans lesquels, en plus de la modification définie dans la revendication 1, l'extrémité 3', les séquences vicinales du site de liaison d'amorce dans le sens 5', seules ou en association, sont bloquées de telle sorte que la région soit incapable de servir de modèle pour l'activité de transcriptase inverse du HTLV-III ;

f) des oligonucléotides modifiés à l'extrémité 5' par addition d'un groupe lipophile, de polylysine, de polyarginine ou d'un autre agent qui accroît la fixation des oligonucléotides, en plus des modifications définies dans la revendication 1,

g) des oligonucléotides modifiés par addition d'un agent d'intercalation, en plus des modifications définies dans la revendication 1,

h) des oligonucléotides comprenant un ou plusieurs groupes réactifs chimiquement capables de provoquer la réticulation de la ou des régions du génome de HTLV-III avec lesquelles ils sont complémentaires, en plus des modifications définies dans la revendication 1,

i) des oligonucléotides comprenant un ou plusieurs groupes réactifs chimiquement capables de cliver la ou les régions du génome de HTLV-III avec lesquelles ils sont complémentaires, en plus des modifications définies dans la revendication 1.

3. Procédé in vitro pour l'inhibition de la réplication du HTLV-III, de l'expression de gènes de HTLV-III ou bien à la fois de cette réplication et de cette expression dans des cellules contenant le HTLV-III, comprenant l'introduction dans les cellules d'un oligodésoxynucléotide modifié suivant la revendication 1 ou la revendication 2.

25

4. Utilisation d'une préparation (ou d'un de ses dérivés modifiés) suivant l'une quelconque des revendications précédentes pour la production d'un médicament destiné à être utilisé dans la chimiothérapie du sida, par exemple dans l'inhibition de la réplication et/ou de l'expression de gènes de HTLV-III dans des cellules de sang périphérique humain (par exemple des lymphocytes T humains) infectées par le HTLV-III.

TARGET SITES

PBS = PRIMER BINDING SITE
sa = SPLICE ACCEPTOR
sd = SPLICE DONOR

AUG(sor)

AUG(tat)

PBS

Frameshift

AUG(gag)

AUG(env)

AUG(3'orf)

cap

sd

sd

sd sa sa

POLYADENYLATION SIGNAL

sa

sa

sd

sa

U5

gag

pol

sor

env

3'orf

R

U3

READING FRAMES

R

LENGTH
(NUCLEOTIDES)

1000  2000  3000  4000  5000  6000  7000  8000  9000

EP 0 402 402 B1